# EUROPÄISCHE PATENTANMELDUNG

(11) **EP 0 650 966 A1**
(43) Veröffentlichungstag der Anmeldung: **03.05.1995**
(21) Anmeldenummer: 94116370.1
(22) Anmeldetag: 18.10.1994
(51) Int. Cl.: C07D 455/06

(54) **Verfahren zur Herstellung eines Benzo(a)chinolizinon-Derivates**

(30) Priorität: 28.10.1993 CH 3252/93
(71) Anmelder: F. HOFFMANN-LA ROCHE AG, CH-4002 Basel (CH)
(72) Erfinder: Spurr, Paul, CH-4125 Riehen (CH)
(74) Vertreter: Poppe, Regina

(57) **Zusammenfassung**

Die Erfindung betrifft ein Verfahren zur Herstellung eines Benzo[a]chinolizinon-Derivates der Formel 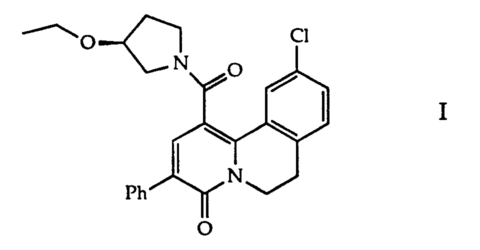
indem man eine Verbindung der Formel 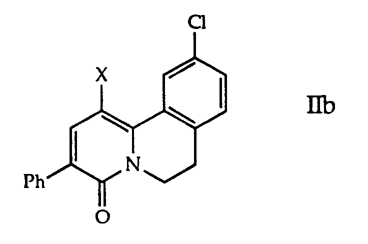
worin X Halogen bedeutet,
in Gegenwart eines Carbonylierungskatalysators und in Gegenwart von (S)-3-Aethoxypyrrolidin oder eines niederen Alkanols oder von Wasser mit Kohlenmonoxid umsetzt; falls ein niederer Alkylester der 10-Chlor-6,7-dihydro-4-oxo-3-phenyl-4H-benzo[a]chinolizin-1-carbonsäure erhalten wurde, diesen in die entsprechende freie Säure überführt; und falls die 10-Chlor-6,7-dihydro-4-oxo-3-phenyl-4H-benzo[a]chinolizin-1-carbonsäure erhalten wurde, diese oder ein reaktives Derivat davon in bekannter Weise mit (S)-3-Aethoxypyrrolidin umsetzt.
Die Verbindung der Formel I besitzt wertvolle pharmakologische Eigenschaften. Sie kann zur Vorbeugung oder Behandlung von Krankheiten verwendet werden, insbesondere zur Behandlung von Schlafstörungen.

## Beschreibung

Die Erfindung betrifft ein Verfahren zur Herstellung eines Benzo[a]chinolizinon-Derivates der Formel

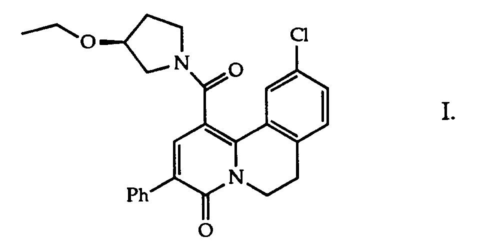

Diese Verbindung der Formel I besitzt wertvolle pharmakologische Eigenschaften. Da sie vor allem eine nichtsedierende, hypnotische und schlaffördernde Wirkung besitzt, kann sie zur Behandlung oder Vorbeugung von Krankheiten verwendet werden, insbesondere zur Behandlung von Schlafstörungen.

Gegenstand der vorliegenden Erfindung ist die Herstellung der Verbindung I, die Herstellung der Zwischenprodukte sowie die Zwischenprodukte der Formeln 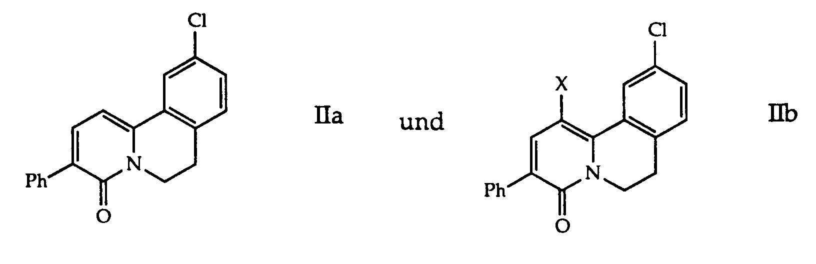
worin X Halogen bedeutet.

Die Verbindung der Formel I an sich, deren Racemat sowie die pharmakologischen Eigenschaften sind bekannt (EP Nr. 183 994 und Nr. 496 274).

Das Benzo[a]chinolizinon-Derivat der Formel I kann erfindungsgemäss hergestellt werden, indem man eine Verbindung der Formel 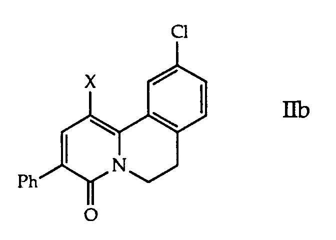
worin X Halogen bedeutet,
in Gegenwart eines Carbonylierungskatalysators und in Gegenwart von (S)-3-Aethoxypyrrolidin oder eines niederen Alkanols oder von Wasser mit Kohlenmonoxid umsetzt; falls ein niederer Alkylester der 10-Chlor-6,7-dihydro-4-oxo-3-phenyl-4H-benzo[a]chinolizin-1-carbonsäure erhalten wurde, diesen in die entsprechende freie Säure überführt; und falls die 10-Chlor-6,7-dihydro-4-oxo-3-phenyl-4H-benzo[a]chinolizin-1-carbonsäure erhalten wurde, diese oder ein reaktives Derivat davon in bekannter Weise mit (S)-3-Aethoxypyrrolidin umsetzt.

Diese Herstellungsvarianten sind in Schema 1 dargestellt: 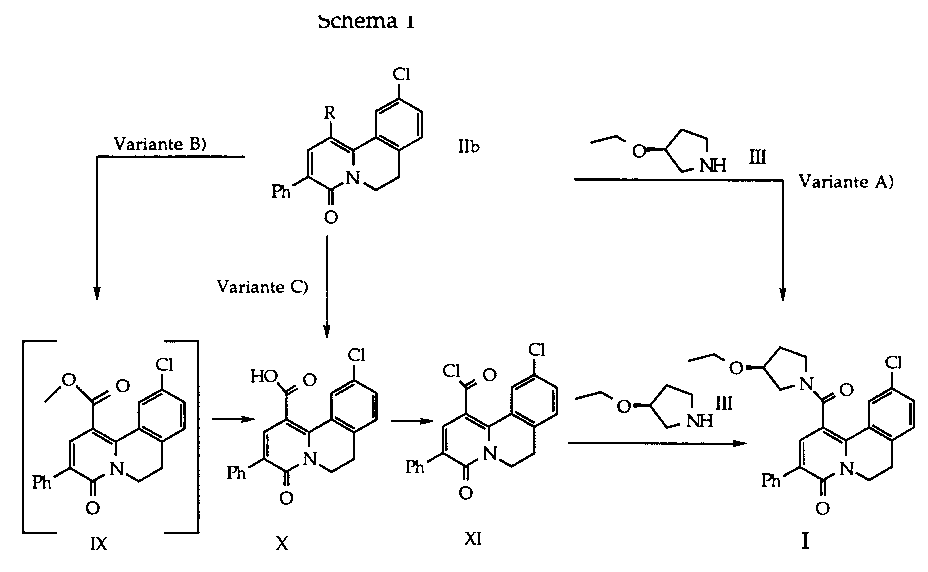
X bedeutet Halogen.

Zweckmässigerweise kann man zur Herstellung der Verbindung I nach Verfahrensvariante A) folgendermassen vorgehen, dabei bedeutet X in der weiteren Beschreibung Brom:
1-Brom-10-chlor-4-oxo-3-phenyl-6,7-dihydro-4H-benzo[a]chinolin (IIb für X = Br), K₂CO₃ und das Pyrrolidin der Formel III werden in einem Lösungsmittel suspendiert. Als Lösungsmittel eignen sich beispielsweise Acetonitril oder Pyridin. Anschliessend wird die Suspension mit einem Katalysator sowie mit 1,3-Bis(diphenylphosphin)propan versetzt. Als Katalysator kann zweckmässigerweise Palladiumacetat verwendet werden. Die Umsetzung erfolgt in einem luftdicht verschlossenen Autoklaven unter Vakuumbedingungen. Der Autoklav wird dann mit Argon entgast und mit ca. 15 bar Kohlenmonoxid beschickt. Die Suspension wird mehrere Stunden bei ca. 100°C gerührt und anschliessend wird das Produkt gereinigt und getrocknet.

Nach Verfahrensvariante B) erfolgt die Herstellung der Verbindung I über mehrere Stufen, wobei der Ester IX nicht isoliert werden muss. Zweckmässigerweise kann man dabei folgendermassen vorgehen:

Die Verbindung IIb für X = Br wird in einem Alkohol, z.B. Methanol, gelöst, mit einem Katalysator, wie beispielsweise Palladiumacetat, versetzt und nach Zugabe einer Base wie KHCO₃ oder KOAc und 1,3-Bis(diphenyl-phosphin)propan in einem Autoklaven, der mit 4-8 bar Kohlenmonoxid beschickt ist, bei ca. 90°C mehrere Stunden umgesetzt.

Der entstandene Ester wird anschliessend mit einer niedrigprozentigen Lauge, z.B. mit KOH, in die entsprechende Säure hydrolysiert. Anschliessend wird die Säure nach an sich bekannten Methoden mit Oxalylchlorid in Anwesenheit von DMAP in Essigester in das entsprechende Säurechlorid überführt. Dieses reaktive Derivat der Formel XI kann anschliessend mit 3-Aethoxypyrrolidin (III) oder dessen Hydrochlorid in Anwesenheit von Triäthylamin in die Verbindung I überführt werden.

Eine weitere Möglichkeit der Herstellung der Verbindung I besteht in der Variante C) des Schemas 1. Hierbei wird die Halogenabgangsgruppe in einer Stufe durch eine Säuregruppe ersetzt. Dabei geht man zweckmässigerweise folgendermassen vor:

Die Verbindung der Formel IIb, worin X vorzugsweise Brom bedeutet, wird in einem Lösungsmittel, beispielsweise DMSO, gelöst, mit Wasser, einem Katalysator, z.B. Palladiumacetat, sowie mit 1,3-Bis(diphenyl-phosphin)propan und mit einer Base, z.B. Kaliumcarbonat, versetzt.

Die Umsetzung erfolgt in einem luftdicht verschlossenen Autoklaven unter ca. 15 bar CO-Atmosphäre. Die Suspension wird mehrere Stunden bei ca. 100°C gerührt. Anschliessend erfolgt die weitere Umsetzung zur Verbindung I nach an sich bekannten Methoden.

Die als Ausgangsstoff verwendete Verbindung III ist bekannt (EP Nr. 496 274) und sie kann beispielsweise durch Alkylierung von (S)-1-Benzyl-3-pyrrolidinol mit einem Aethylhalogenid, wie Aethylbromid oder Aethyljodid, in Gegenwart einer Base und anschliessendem Abspalten der Benzylgruppe durch katalytische Hydrogenolyse hergestellt werden. (S)-1-Benzyl-3-pyrrolidinol ist eine in der Literatur beschriebene Verbindung; vgl. J. Med. Chem. 29, 2504-2511 (1986) und Synth. Comm. 15, 587-598 (1985).

Die Verbindungen der Formel IIa und IIb sind neu und können gemäss Schema 2 hergestellt werden: 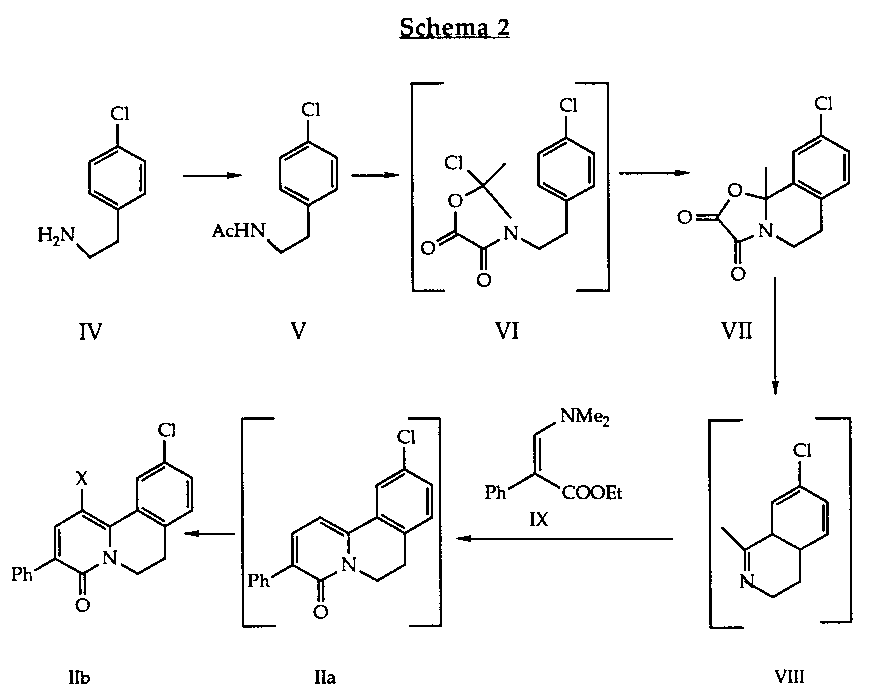
Dabei bedeutet X Halogen.

Zweckmässigerweise geht man bei der Herstellung der Verbindungen IIa bzw. IIb folgendermassen vor:
Die Acylierung der Verbindung IV kann mit Acetanhydrid erfolgen. Als Lösungsmittel bei dieser exothermen Reaktion, die bei Raumtemperatur durchgeführt wird, ist Toluol besonders geeignet. Die Essigsäure, die bei der Acylierung als Nebenprodukt anfällt, kann anschliessend durch eine azeotrope Destillation entfernt werden. Das Amid V kann auch durch eine Amidierung von Amin IV mit Methylformiat, Ethylacetat oder Isopropylacetat hergestellt werden.

Die erhaltene Verbindung V wird nach Umsetzung mit Oxalylchlorid in Dichlormethan bei Raumtemperatur in das Zwischenprodukt VI überführt. Dieses kann ohne Zwischenisolierung in Gegenwart einer Lewis-Säure zur Verbindung VII überführt werden. Am geeignetsten erweist sich die Kombination FeCl₃/CH₂Cl₂. Aber auch die folgenden Lewis-Säuren sind möglich: BF₃·OEt₂; AlCl₃; TiCl₄ oder SnCl₄ (J. Org. Chem., 56, 6034 [1991]).

Die weitere Umsetzung der Verbindung VII zur Verbindung IIa kann auf unterschiedliche Weise erfolgen. Zweckmässig erweist sich eine Thermolyse der Verbindung VII mit Essigsäure bei Temperaturen zwischen 100-120°C zu den Dihydroisochinolinen der Formel VIII. Für eine weitere Umsetzung braucht diese Verbindung nicht isoliert werden. Die in der Literatur noch nicht beschriebene Ringschlussbildung der Verbindung VIII mit einem Aminoacrylat der Formel IX erfolgt zweckmässigerweise in einem polaren, sauren Medium, das gleichzeitig als Katalysator und als Lösungsmittel geeignet ist, beispielsweise in Eisessig bei ca. 100°C. Eine erfolgreiche Reaktion ist jedoch auch möglich in einem polaren neutralen Medium wie DMF bei höheren Temperaturen. Die Umsetzungen erfolgen innerhalb einiger Stunden, und wird die Verbindung IIa isoliert, erhält man Ausbeuten von 70 bis 80%.

Eine weitere Möglichkeit der Herstellung der Verbindung IIa, ausgehend von VII, besteht in der Behandlung mit konzentrierter Schwefelsäure in rückfliessendes Methanol, anschliessender Isolierung von Dihydroisochinolin sowie Umsetzung mit dem Aminoacrylat IX die, wie oben beschrieben, erfolgen kann.

Es ist auch möglich, alle Reaktionspartner gleichzeitig einzusetzen. Dazu versetzt man die Verbindung der Formel VII mit dem Aminoacrylat IX. Als Lösungsmittel verwendet man zweckmässigerweise Essigsäure. Es wird mehrere Stunden gerührt, wobei die Reaktionstemperatur ca. 100°C beträgt. Anschliessend kann die entstandene Verbindung IIa mit oder ohne Zwischenisolierung halogeniert werden.

Als Abgangsgruppe für die Herstellung der Verbindung I ist Brom am besten geeignet. Deshalb wird im folgenden eine mögliche Bromierungsvariante näher beschrieben:

Zweckmässigerweise wird die sich in Lösung befindliche oder isolierte Verbindung IIa mit N-Br-Succinimid behandelt. Als Lösungsmittel kann man Essigsäure, CH₂Cl₂ oder MeCN verwenden. Die Bromierung erfolgt bei Temperaturen zwischen Raumtemperatur und 95°C. Die Ausbeute der Verbindung IIb ist quantitativ.

Die Anwesenheit von Nebenprodukten, die aus der Reaktion der Verbindungen der Formeln VIII und IX hervorgegangen sind, haben für den Verlauf der Bromierung keine Bedeutung. Die Bromverbindung der Formel IIb fällt durch Zugabe von Wasser zur heissen Reaktionslösung beim Abkühlen auf Raumtemperatur aus. Die öligen Nebenprodukte verbleiben in Lösung. Das erfindungsgemässe Verfahren, einschliesslich der beschriebenen Verfahrensvarianten, ermöglicht es, die pharmakologisch wirksame Verbindung der Formel I in über dreimal so hoher Ausbeute zu erhalten wie das bekannte Verfahren, beschrieben in Helv. Chim. Acta 73, 763 (1990).

Das war auf Grund der vielen Verfahrensschritte, die teilweise ohne Zwischenisolierung der entstandenen Zwischenprodukte erfolgten, nicht zu erwarten.

Ein weiterer Vorteil des neuen Verfahrens besteht darin, dass die Zwischenstufen über die Bildung von Isomeren oder von einem Isothiocyanat, die aus ökologischen und toxischen Gründen ein Verfahren unwirtschaftlich gestalten, nicht benötigt werden.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

### 1. Herstellung der Zwischenprodukte der Formeln IIa und IIb

### a) 2-(4-Chlorphenyl)-ethylacetamid (V)

155,6 g (1 Mol) 2-(4-Chlorphenyl)-äthylamin (IV) wurden in 500 ml Toluol gelöst und mit 107,2 g (1,05 Mol) Acetanhydrid innerhalb von 0,5 Stunden versetzt. Anschliessend wurde 0,25 Stunden bei 80° gerührt. Die Lösung wurde eingeengt, in 250 ml Toluol aufgenommen und nach Trocknen bei 40°/0,1 mbar/1,5 h erhielt man ein Oel, das langsam fest wurde.

Ausbeute: quantitativ, Smp. 93-94° (Toluol/Hexan).

### b) (R,S)-9-Chlor-10b-methyl-2,3,6,10b-tetrahydro-5H-oxazolo[2,3-a]isochinolin-2,3-dion (VII)

204,9 g (1 Mol) 2-(4-Chlorphenyl)-äthylacetamid (V) wurden in 2000 ml Dichlormethan gelöst und innerhalb von 0,75 Stunden mit 94,5 ml (1,1 Mol) Oxalylchlorid versetzt. Die Lösung wurde noch 0,5 Stunden bei 30° gerührt und anschliessend auf 5° abgekühlt. Danach wurde die Lösung portionsweise innerhalb von 0,5 Stunden mit 194,7 g (1,2 Mol) FeCl₃ versetzt, wobei die Temperatur auf 25° anstieg. Bei dieser Temperatur wurde 16 Stunden gerührt. Die entstandene schwarze Suspension wurde anschliessend mit 1000 ml 4N HCl versetzt und noch 1 Stunde nachgerührt. Die organische Phase wurde danach abgetrennt und einmal mit 500 ml Wasser gewaschen. Die Wasserphase wurde zweimal mit je 500 ml Dichlormethan extrahiert. Die organischen Extrakte wurden vereinigt und eingeengt. Es entstand ein hellbrauner Feststoff.

Ausbeute: 96%, Smp. 169° (CH₂Cl₂, Zers. zu (VIII)).

Als Alternative für die Aufarbeitung kann nach der Behandlung des Reaktionsgemisches mit HCl das Lösungsmittel eingeengt werden. Das ausgefallene Produkt wird abfiltriert und mehrmals mit Wasser gewaschen.

Variante a) zur Herstellung von (IIb)

### c) 7-Chlor-3,4-dihydro-1-methylisochinolin (VIII)

Eine Lösung von 248,1 g (1,0 Mol) (R,S)-9-Chlor-10b-methyl-2,3,6,10b-tetrahydro-5H-oxazolo[2,3-a]isochinolin-2,3-dion (VII) und 2000 ml Methanol wurde innerhalb von 0,5 Stunden mit 150 ml (2,8 Mol) konzentrierter Schwefelsäure versetzt. Dabei stieg die Temperatur auf ca. 45°. Anschliessend wurde die braune Suspension 24 Stunden am Rückfluss gekocht, bis die Lösung eine klare dunkelbraune Färbung annahm. Dann wurde das Lösungsmittel eingeengt und mit 1000 ml Wasser versetzt. Die wässrige Lösung wurde dreimal mit insgesamt 1500 ml Aethylacetat extrahiert und die vereinigten organischen Extrakte wurden zweimal mit 250 ml Wasser gewaschen. Die Wasserphasen wurden vereinigt und mit 500 ml 25%iger NH₄OH auf pH = 9 eingestellt. Die ölige obere Phase wurde dreimal mit je 500 ml Dichlormethan extrahiert. Die vereinigten organischen Phasen wurden zweimal mit je 250 ml Wasser gewaschen und filtriert. Das Filtrat wurde eingeengt und es entstand ein dunkel-gelb-grünes Oel, das bei 45°/0,1 mbar getrocknet wurde.

Ausbeute: 164.7 g, 92%. Nach einer Weile entstanden Plätzchen mit Smp. 40-42°.

### d) 10-Chlor-4-oxo-3-phenyl-6,7-dihydro-4H-benzo[a]chinolin (IIa)

89,8 g (0,5 Mol) 7-Chlor-3,4-dihydro-1-methyl-isochinolin (VIII) und 115,1 g (0,525 Mol) Aethyl-E/Z-2-phenyl-3-(dimethylamino)acrylat wurden in 600 ml Eisessig gelöst und innerhalb von 0,33 Stunden auf 95° (Oelbad) erhitzt. Anschliessend wurde 3 Stunden gerührt. Es entstand eine dunkelbraune Lösung.

### e} 1-Brom-10-chlor-4-oxo-3-phenyl-6,7-dihydro-4H-benzo[a]chinolin (IIb)

Die in Beispiel 1d) hergestellte Lösung wurde bei 90° mit 111,2 g (0,625 Mol) N-Bromsuccinimid versetzt und unter Erwärmen 1 Stunde gerührt. Die Kristallisation des Zielproduktes erfolgte durch Zugabe von 150 ml Wasser. Innerhalb von 0,75 Stunden wurde auf 20° abgekühlt und anschliessend noch 0,5 Stunden gerührt. Das ausgefallene Produkt wurde filtriert und anschliessend mit 200 ml einer Lösung, die aus Essigsäure und Wasser im Verhältnis von 1:1 bestand, und dreimal mit kaltem t-Butyl-methylether gewaschen. Nach dem Trocknen (60°/40 mbar/4 h) erhielt man das Bromid als gelben kristallinen Feststoff. Ausbeute: 138,5 g = 72%, Smp. 196-197° (AcOH/H₂O).

Variante b) zur Herstellung von (Ib)

### 1-Brom-10-chlor-4-oxo-3-phenyl-6,7-dihydro-4H-benzo[a]chinolin (IIb)

Eine Lösung aus 5,03g (20 mMol) (R,S)-9-Chlor-10b-methyl-2,3,6,10b-tetrahydro-5H-oxazolo[2,3-a]isochinolin-2,3-dion (VII) und 4,40 g (20 mMol) Aethyl-E/Z-2-phenyl-3-(dimethylamino)acrylat (IX) in 25 ml Essigsäure wurde 4 Stunden bei 100° gerührt. Ohne Zwischenisolierung der entstandenen Verbindung 10-Chlor-4-oxo-3-phenyl-6,7-dihydro-4H-benzo[a]chinolin (IIa) versetzte man diese Lösung innerhalb von 0,25 Stunden portionsweise mit 4,45 g (25 mMol) N-Bromsuccinimid und rührte anschliessend die entstandene braune Reakfionslösung 1 Stunde bei 100°.

Nach Zugabe von 5 ml Wasser und Abkühlung des Reaktionsgemisches auf Raumtemperatur kristallisierte die Verbindung in gelben Nadeln aus. Der Rückstand wurde filtriert und das Produkt zweimal mit je 10 ml wässriger Essigsäure (1:1) und einmal mit 5 ml t-Butylmethyläther gewaschen. Die Ausbeute betrug 5,76 g (75%).

Variante c) zur Herstellung von (IIb)

### 1-Brom-10-chlor-4-oxo-3-phenyl-6,7-dihydro-4H-benzo[a]chinolin (IIb)

Eine Lösung von 5,03 g (20 mMol) (R,S)-9-Chlor-10b-methyl-2,3,6,10b-tetrahydro-5H-oxazolo[2,3-a]isochinolin-2,3-dion (VII) in 25 ml Essigsäure wurde 4 Stunden bei 120° gerührt. Es entstand 7-Chlor-3,4-dihydro-1-methyl-isochinolin als braune Reaktionslösung (VIII). Es wurde auf 100° abgekühlt und mit 4,40 g (20 mMol) Aethyl-E/Z-2-phenyl-3-(dimethylamino)acrylat (IX) versetzt. Anschliessend wurde bei 100° 3 Stunden gerührt und die Reaktionslösung mit 4,45 g (25 mMol) N-Bromsuccinimid versetzt. Die Zugabe erfolgte portionsweise innerhalb 0,25 Stunden. Es wurde anschliessend 1 Stunde bei 100° gerührt.

Nach Zugabe von 5 ml Wasser und Abkühlung des Reaktionsgemisches auf Raumtemperatur kristallisierte die Verbindung in gelben Nadeln aus. Der Rückstand wurde abfiltriert und das Produkt zweimal mit je 10 ml wässriger Essigsäure (1:1) und einmal mit 5 ml t-Butylmethyläther gewaschen. Die Ausbeute betrug 6,03 g (76%).

### 2. Herstellung von (S)-1-[(10-Chlor-6,7-dihydro-4-oxo-3-phenyl-4H-benzo[a]chinolizin-1-yl)carbonyl]-3-äthoxypyrrolidin I

### 2.1.a) 10-Chlor-4-oxo-3-phenyl-6,7-dihydro-4H-benzo[a]chinolizin-1-carbon-säure (X) (Variante B)

Eine Suspension von 38,67 g (100 mMol) 1-Brom-10-chlor-4-oxo-3-phenyl-6,7-dihydro-4H-benzo[a]chinolin (IIb) und 500 ml Methanol wurde in einem Glasautoklaven mit 0,011 g (0,05 mMol) Palladiumacetat, 0,025 g (0,06 mMol) 1,3-Bis(diphenylphosphin)propan sowie mit 20 g (200 mMol) KHCO₃ versetzt. Im Autoklaven wurde ein Druck von 0,1 bar eingestellt und unter Argonatmosphäre entgast. Die Entgasung wurde zweimal wiederholt. Bei 0,1 bar wurde der Autoklav mit 4 bar Kohlenmonoxid beschickt. Die Suspension wurde 20 Stunden bei 90° (Oelbad 100°) bei 700 U/min. gerührt. Anschliessend wurde die klare gelbe Lösung auf 20-25° abgekühlt und das überschüssige Kohlenmonoxid abgelassen. Beim Abkühlen entstand eine dickflüssige Suspension des Esters IX, die gerührt und noch dreimal entgast wurde. In einer anderen Apparatur wurde das Reaktionsemisch mit Hilfe von einer Lösung von 14,0 g KOH (0,25 Mol) in 500 ml Wasser sowie 50 ml Methanol umgespült und 4 Stunden bei 75° am Rückfluss (Oelbad 90°) gekocht. Die olivegrüne Lösung wurde auf 50° abgekühlt, durch ein Florosilposter filtriert und der Rückstand mit 50 ml Methanol gewaschen. Das Filtrat wurde eingeengt und gleichzeitig mit 500 ml Wasser bei 40° getauscht. Die Wasserphase wurde zweimal mit je 250 ml Aethylacetat gewaschen und die vereinigten Wasserphasen wurden mit 1% KOH extrahiert. Die organische Phase wurde verworfen. Die Wasserphase wurde auf 5° abgekühlt und mit 40 ml konzentrierter HCl auf pH 1 eingestellt. Danach wurde 0,25 Stunden gerührt, die entstandene Säure abfiltriert und portionsweise mit 150 ml Wasser gewaschen. Die Trocknung und Isolierung des entstandenen weissen Pulvers erfolgt bei 50°/0,1 mbar/7 h. Ausbeute 98%, Smp. 285-286° (Zers.).

### 2.1.b) (S)-1-[(10-Chlor-6,7-dihydro-4-oxo-3-phenyl-4H-benzo[a]chinolizin-1-yl)carbonyl]-3-äthoxypyrrolidin (I)

Eine Suspension von 8,79 g (25 mMol) 10-Chlor-4-oxo-3-phenyl-6,7-dihydro-4H-benzo[a]chinolizin-1-carbonsäure (X) in 125 ml Aethylacetat versetzte man mit 0,076 g (0,625 mMol) Dimethylaminopyridin und 3,49 g (27,5 mMol) Oxalylchlorid. Dann wurde 3 Stunden bei 60° gerührt. Die gelbe Lösung wurde eingeengt und der Rückstand in 125 ml Toluol aufgenommen. Anschliessend wurde entweder mit 5,26 g (52 mMol) Triäthylamin und 3,94 g (26 mMol) (S)-3-Aethoxypyrrolidin-hydrochlorid oder mit 2,63 g (26 mMol) Triäthylamin und 3,0 g (26 mMol) (S)-3-Aethoxypyrrolidin umgesetzt und 4 Stunden gerührt. Anschliessend wurde die Lösung nacheinander mit 100 ml Wasser, 100 ml 1N HCl, 100 ml 0,5N KHCO₃ und dann wieder mit 100 ml Wasser gewaschen. Die einzelnen Wasserphasen wurden mit 100 ml Toluol extrahiert und die vereinigten organischen Extrakte wurden mit 20 g Na₂SO₄ getrocknet. Der nach Filtrierung und Einengung erhaltene Rückstand wurde bei 50°/0,1 mbar/2 h getrocknet. Nach Reinigung durch Umkristallisation aus Aceton/Wasser und Aceton/Hexan erhielt man die Verbindung IIb in 85%iger Ausbeute, bezogen auf die Säure. Smp. 133-134°.

### 2.2. (Variante C)

In einem Autoklaven versetzte man eine Suspension von 3,86 g (10 mMol) 1-Brom-10-chlor-4-oxo-3-phenyl-6,7-dihydro-4H-benzo[a]chinolin (IIb) in 25 ml Acetonitril mit 1,21 g (10,5 mMol) (S)-3-Aethoxypyrrolidin (III), 0,022 g (0,1 mMol) Palladiumacetat, 0,062 g (0,15 mMol) 1,3-Bis(diphenylphosphin)propan und 6,91 g (50 mMol) Kaliumcarbonat. Der Autoklav wurde luftdicht verschlossen und auf 0,1 bar eingestellt. Die Rührergeschwindigkeit betrug 700 U/min. Der Entgasungsvorgang wurde zweimal mit Argon wiederholt. Dann wurde wieder auf 0,1 bar eingestellt. Anschliessend wurde der Autoklav bei ca. 15 bar mit Kohlenmonoxid beschickt und die Suspension 24 Stunden bei 100° gerührt. Bei dieser Temperatur betrug der CO-Druck ca. 20 bar. Die Lösung wurde anschliessend auf eine Temperatur von ca. 20° gekühlt, das überschüssige CO wurde abgeblasen. Die entstandene gelbe Suspension wurde mit 50 ml Aethylacetat und 50 ml Wasser versetzt. Die wässrige Phase wurde abgetrennt und mit 25 ml Aethylacetat extrahiert. Die vereinigten organischen Phasen wurde über Natriumsulfat getrocknet, filtriert und eingeengt. Es entstand ein beige gefärbter Feststoff, der mittels Chromatographie (EtOAc/Hexan) gereinigt wurde. Ausbeute: 3,98 g, 89%.

### 2.3. (Variante A)

Eine Lösung von 3,86 g (10 mMol) 1-Brom-10-chlor-4-oxo-3-phenyl-6,7-dihydro-4H-benzo[a]chinolin (Vb) in 45 ml Dimethylsulfoxid versetzte man mit 5 ml Wasser, 0,022 g (0,1 mMol) Palladiumacetat, 0,062 g (0,15 mMol) 1,3-Bis(diphenylphosphin)propan und 2,0 g (20 mMol) KHCO₃ in einem Autoklaven. Der Autoklav wurde luftdicht verschlossen und auf 0,1 bar eingestellt. Die Rührergeschwindigkeit betrug 700 U/min. Der Entgasungsvorgang wurde zweimal wiederholt und anschliessend wurde wieder auf 0,1 bar eingestellt und mit ca. 15 bar Kohlenmonoxid beschickt. Die Suspension wurde bei 100° 20 Stunden gerührt. Bei dieser Temperatur betrug der CO-Druck ca. 20 bar. Die Lösung wurde anschliessend auf eine Temperatur von 20° gekühlt und das überschüssige CO abgeblasen. Die entstandene gelbe Suspension wurde mit 0,6 g NaOH in 150 ml Wasser versetzt, gefiltert und der Filterrückstand wurde mit 50 ml Wasser versetzt. Das Filtrat wurde dreimal mit je 50 ml Aethylacetat gewaschen. Die gewaschenen und vereinigten Wasserphasen wurden auf 5° gekühlt und mit 5 ml konzentrierter HCl auf pH 1 eingestellt. Der entstandene Niederschlag wurde abfiltriert, zweimal mit 50 ml Wasser gewaschen und getrocknet. Ausbeute 3,17 g (90%).

Die weitere Umsetzung zu Verbindungen der Formel I erfolgte wie im Beispiel 2.1.b) beschrieben.

## Patentansprüche

1. Verfahren zur Herstellung eines Benzo[a]chinolizinon-Derivates der Formel 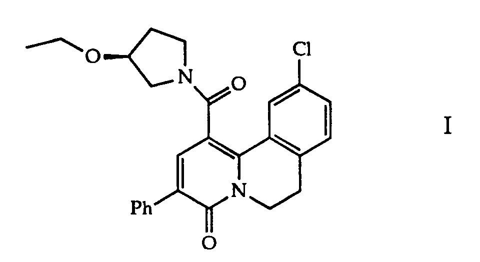 dadurch gekennzeichnet, dass man eine Verbindung der Formel 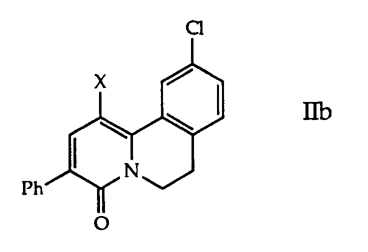 worin X Halogen bedeutet,
in Gegenwart eines Carbonylierungskatalysators und in Gegenwart von (S)-3-Aethoxypyrrolidin oder eines niederen Alkanols oder von Wasser mit Kohlenmonoxid umsetzt; falls ein niederer Alkylester der 10-Chlor-6,7-dihydro-4-oxo-3-phenyl-4H-benzo[a]chinolizin-1-carbonsäure erhalten wurde, diesen in die entsprechende freie Säure überführt; und falls die 10-Chlor-6,7-dihydro-4-oxo-3-phenyl-4H-benzo[a]chinolizin-1-carbonsäure erhalten wurde, diese oder ein reaktives Derivat davon in bekannter Weise mit (S)-3-Aethoxypyrrolidin umsetzt.

2. Benzo[a]chinolin-Derivate der Formeln 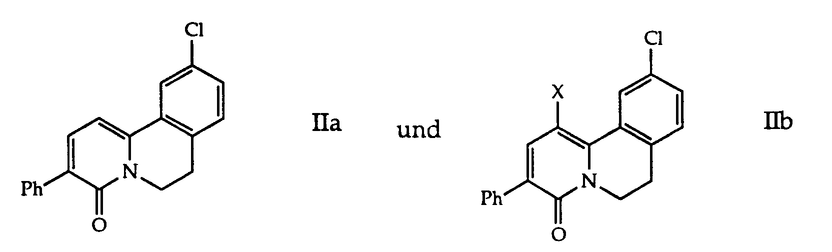 worin X Halogen bedeutet.

3. 10-Chlor 4-oxo-3-phenyl-6,7-dihydro-4H-benzo[a]chinolin.

4. 1-Brom-10-chlor-4-oxo-3-phenyl-6,7-dihydro-4H-benzo[a]chinolin.

5. Verfahren zur Herstellung der Verbindung der in Anspruch 2 angegebenen Formel IIa, dadurch gekennzeichnet, dass man die Verbindung der Formel 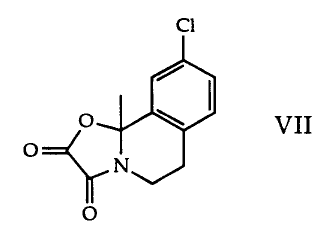 zur Verbindung der Formel 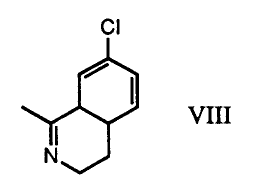 hydrolysiert und diese dann mit der Verbindung der Formel 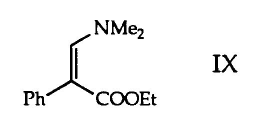 umsetzt, oder dass man die Verbindung der Formel VII direkt mit der Verbindung der Formel IX umsetzt.

6. Verfahren zur Herstellung einer Verbindung der in Anspruch 2 angegebenen Formel IIb, dadurch gekennzeichnet, dass man die Verbindung der in Anspruch 2 angegebenen Formel IIa mit einem Halogenierungsmittel behandelt.
